(19) Europäisches Patentamt
European Patent Office
Office européen des brevets

(11) **EP 3 576 637 B1**

(12) **EUROPEAN PATENT SPECIFICATION**

(45) Date of publication and mention of the grant of the patent:
**22.11.2023 Bulletin 2023/47**

(21) Application number: **18705560.3**

(22) Date of filing: **05.02.2018**

(51) International Patent Classification (IPC):
***A61B 17/00*** (2006.01)

(52) Cooperative Patent Classification (CPC):
**A61B 17/0057; A61B 17/00491;** A61B 2017/00575;
A61B 2017/00597; A61B 2017/0061;
A61B 2017/00623; A61B 2017/0065;
A61B 2017/00884

(86) International application number:
**PCT/EP2018/052765**

(87) International publication number:
**WO 2018/141951 (09.08.2018 Gazette 2018/32)**

(54) **DEVICE FOR SEALING A MEMBRANE**

VORRICHTUNG ZUM VERSCHLUSS EINER MEMBRAN

DISPOSITIF POUR SCELLER UNE MEMBRANE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.02.2017 EP 17154743**
**29.09.2017 EP 17194141**
**06.10.2017 EP 17195183**

(43) Date of publication of application:
**11.12.2019 Bulletin 2019/50**

(73) Proprietor: **Universität Zürich**
**8006 Zürich (CH)**

(72) Inventors:
• **DEVAUD, Yannick**
**1700 Fribourg (CH)**
• **MILLERET, Vincent**
**8057 Zurich ZH (CH)**
• **ZIMMERMANN, Roland**
**8044 Gockhausen ZH (CH)**
• **EHRBAR, Martin**
**9500 Wil SG (CH)**
• **OCHSENBEIN, Nicole**
**8049 Zurich ZH (CH)**

(74) Representative: **Schulz Junghans**
**Patentanwälte PartGmbB**
**Großbeerenstraße 71**
**10963 Berlin (DE)**

(56) References cited:
**EP-A1- 3 300 669**     **WO-A1-2013/009872**
**WO-A2-2011/057282**     **US-A1- 2007 276 435**
**US-A1- 2008 058 710**

**Description**

**TECHNICAL FIELD AND BACKGROUND**

Embodiments disclosed herein relate in general to devices and methods for sealing a membrane such as a fetal membrane.

The fetal membrane is a relatively thin tissue layer having a thickness of a few hundred microns that surrounds the fetus during development. The fetal membrane holds amniotic fluid and creates a physical barrier, for example, to protect the fetus from infections and to provide paracrine signaling between the mother and the embryo. Intact fetal membranes are of central importance to a pregnancy. Preterm pre-labor rupture of fetal membranes (PPROM) initiates delivery in most cases, which is a serious complication in early pregnancy. Indeed, preterm birth carries a big risk of complication ranging from incurable diseases decreasing life quality and/or expectancy and range from cerebral palsy, impaired cognitive skills, vision problems, hearing problems, behavioral, psychological as well as chronic health issues to morbidity.

With advances in fetal diagnosis and therapies, fetoscopy has become a clinical routine. This surgical technique aims at treating potentially life-threatening diseases during pregnancy by invasive intervention into the amniotic cavity. Example medical indications include twin-to-twin transfusion syndrome (TTTS), discordant monochorionic twins with a lethal anomaly, reverse arterial perfusion (TRAP) as well as severe congenital diaphragmatic hernia (CDH). Depending on the type of intervention, the injury or perforation created by the fetoscopy can induce iatrogenic PPROM (iPPROM) in 6 to 45% of the cases. This unsolved clinical problem compromises benefits of the surgical intervention and blocks tremendously the advances in treatment technologies.

Since fetal membranes have a limited spontaneous healing potential, the application of natural and synthetic materials to close the defect has been studied in the past. Such approaches have failed to prove clinical relevance due to inadequate material stability or to the absence of healing-inducing factors.

The description above is presented as a general overview of related art in this field and should not be construed as an admission that any of the information it contains constitutes prior art against the present patent application.

**GENERAL DESCRIPTION OF THE INVENTION**

Embodiments of the present invention are aimed at overcoming some of the disadvantages associated with the application of, e.g., polymeric biomimetic gluing material such as "mussel glue" for the sealing of fetal membranes. The potential in the use of such polymeric biomimetic gluing material for the sealing of membranes was described in Lee et al., Nature 2007, 448. 338-41, and further in Bilic et al., American Journal of Obstetrics and Gynecology 2010, 202. 85 e1-9. Further, Haller et al. disclose in Acta biomaterialia 2012, 8. 4365-70 that mussel glue is non-toxic, tightly adheres to fetal membranes and withstands substantial deformation and/or pressure of fetal membranes.

However, some of the problems that were encountered in all the above-noted studies was the difficulty to deliver the gluing material to the desired location and the lack of a reproducible and robust method to apply the gluing material at the desired location for closing the perforated membrane. For example, EP3300669 discloses a membrane closure device for closing a perforation in a membrane, wherein said device comprises a patch that is selectively expandable from a narrow to an expanded configuration and having a concave surface defining a retention cavity for receiving glue. The device comprises a patch delivery mechanism and fastener delivery mechanism operable to contain glue and further operable to deliver the glue contained therein from the proximal side to the distal side of the membrane for fastening the patch in the expanded configuration, by the glue, to the distal side of the membrane to seal the perforation. In the same field of endeavor US 2008/058710 A1 teaches a surgical device for closing a perforation in a membrane using a tubular member, a ring made of flexible material, a resilient membrane, and a pusher member.

Devices according to embodiments of the present invention may overcome at least some of the above-noted problems. The invention is defined by appended claim 1. Specific embodiments are set forth in the dependent claims.

The following description of devices of the invention is given with reference to particular examples, with the understanding that such devices are not limited to these examples.

The disclosed embodiments relate to membrane closure devices and methods for repairing a perforated (also: ruptured) membrane. Such membrane can, for example, include a fetal membrane, vascular membrane, cardiovascular membrane and/or a tympanic membrane. Devices and methods described herein may be applicable to any type of perforated membrane or layer, whether living or non-living.

According to some embodiments, the membrane closure device is configured or adapted to allow, for example, targeted delivering of glue to patch for the instantaneous gluing thereof to a fetal membrane, without requiring additional mechanical fasteners or elements such as tackers, clamps, staplers, sutures and/or the like, which could perforate, puncture, rupture, or otherwise damage the membrane. The devices of the present invention allow the sealing of a membrane with a single patch. The disclosed methods may thus include the delivery and/or use of a single patch for the sealing of a membrane from its underside. Embodiments of the devices and methods allow one-sided sealing of a membrane.

In an embodiment, the patch does not require an adhesive layer, since a glue is provided separate from the patch for fastening thereof to the membrane. In other words, the patch may be free of an adhesive layer during its delivery to the perforation site. Optionally, the glue may comprise a bioactive agent. Optionally, the patch may comprise a bioactive agent. Optionally, only the glue may comprise a bioactive agent.

According to some embodiments, the membrane closure device for closing a perforation in a membrane, comprises a patch delivery mechanism for delivering and deploying one or more (e.g., expandable) patches to a perforation site of the membrane. The membrane closure device further comprises a fastener delivery mechanism for delivering a fastener to the perforation site for fastening the one or more expandable patches, to the membrane to seal the perforation in the membrane. Such fastener can be, for example, a sealant, a sealant-inducing material, healing-inducing matter, a plug, and/or a weld. The thickness of the patch can range, for example, from about 50 micron to about 1mm. Optionally, the fastener delivery mechanism may include the patch delivery mechanism, or vice versa. Optionally, at least a portion of the fastener delivery mechanism may constitute the patch delivery mechanism, or vice versa.

According to some embodiments, the fastener delivery mechanism comprises one or more syringes barrel operable to receive glue and which are further operable to receive one or more respective plungers for pushing the glue or glue components through corresponding one or more distal ends of the one or more syringe barrel into a retention cavity formed by the patch when in the expanded configuration.

The membrane closure device may be operable to access the perforation site from a proximal side thereof for sealantly securing the patch with glue to the underside or distal side of the perforation site. The membrane closure device may be operable to sealantly secure the patch to the underside of the perforation site without requiring the use of tackers, staples and/or other elements that would puncture or otherwise damage the fetal membrane. In other words, membrane closure device allows securing the patch to the perforation in a "tacker/staple-free manner". Moreover, the membrane closure device allows the user to operably position the patch without requiring a field-of-view (FOV) of the perforation from the membrane underside, so that, for example, access for fetoscopy and for delivering the patch may be gained via the same perforation. Optionally, no imaging techniques (e.g., Ultrasound imager) may have to be employed while applying the patch to sealantly secure the patch to the perforation site. Hence, only one perforation may be needed to allow conducting fetoscopy and to seal the underside of the perforation by employing the membrane closure device. For example, the membrane closure device was inserted immediately after fetoscopy through the exact same catheter used for the intervention in order to ensure precise placement at the site of puncture. Aspects of embodiments may pertain to a fetoscope that is configured to comprise the membrane closure device.

Optionally, one or more expandable bodies (e.g., balloons) may be employed to allow stabilizing the fetal membrane between such expandable body and the expanded patch while pulling the patch against the fetal membrane. The one or more expandable bodies may allow applying a counterforce in a distal direction while, at the same time, pulling the patch against the fetal membrane in a proximal direction. The membrane closure device may be operable such that the counterforce increases responsive to an increase in the pulling force. The patch and/or the expandable units are selectively expandable.

Merely to simplify the discussion that follows, without be construed limiting, the terms "fastener", "glue", and "sealant" may herein be used interchangeably.

The membrane closure device comprises a tube-shaped body (also: tube) having a distal and a proximal end. The tube-shaped body defines internal to the tube a patch delivery passageway (also: operating channel) for delivering the expandable patch in a narrow (e.g., crimped or folded) configuration, via the tube, from the proximal to the distal end for attaching the expandable patch, using the fastener, in an expanded configuration to a surface (e.g., distal surface) of the membrane.

The relative positional term "proximal" as used herein refers to a location that is situated closer from a user of the device during normal use of the device than a location that is "distal" to the user during such normal use. For example, a proximal end is an end point of the tube situated closest to the user of the device, and a distal end is an endpoint situated farthest from the user.

An outer membrane surface of a membrane is a surface that has to be engaged first by the device for gaining access to, e.g., an amniotic cavity enclosed by the membrane. An inner membrane surface may refer to the surface that is in direct contact with the content of, e.g., the amniotic cavity and that can be engaged only after engagement of the device with the outer surface. It is noted that the outer and inner membrane surfaces do not necessarily have to pertain to amniotic membranes, but may also pertain to other membranes or tissue of, e.g., mammals.

Merely to simplify the discussion that follows, the term "proximal membrane surface" may herein also be referred to as "outer surface" or "upper surface". Correspondingly, the term "inner membrane surface" may herein also be referred to as "inner surface" or "undersurface".

In the context of the present disclosure, a longitudinal direction of the tube extends from its proximal end along a longitudinal axis to the distal end. The latitudinal direction is perpendicular to the longitudinal axis, along a latitudinal axis of the tube.

When in the folded configuration, the patch can slide (e.g., by applying a pushing and/or pulling force) through the tube along its longitudinal axis from the distal to the proximal end. Upon exiting the tube's distal end, the patch is set in the expanded configuration (e.g., by expanding in latitudinal direction) for covering the perforation.

The term "user" as used herein may include, for example, a physician or medical doctor. The term "patient" as used herein refers to the person that is subjected to procedures for closing a perforated membrane in the body of the patient.

The terms cited above denote also grammatical variations thereof.

Merely, for brevity and clarity, and therefore without be construed limiting, in the present disclosure a membrane closure device is herein exemplified for use during a minimally invasive surgery (MIS).

The outer diameter of the portions of the tube-shaped body perforating the membrane for, e.g., fetoscopy - which may be performed at a gestational stage ranging, e.g., from week 17 to week 27 - may range, for example, from 1.7 mm to 4 mm. The diameter may depend, inter alia, on the fetoscopy procedure required to be performed. Fetoscopy procedures can include, for example, placental laser surgery, delivering a balloon into the trachea to occlude the airway for the treatment of congenital diaphragmatic hernia, shunting, and/or any other fetal treatment. The patch in the folded configuration is adapted or configured to slidably fit into the patch delivery passageway, which is internal to the tube-shaped body. In the expanded configuration, the patch's diameter can for example be at least 2, 3, 4, or 5 times larger than the tube-shaped body's outer diameter. The patch's diameter, in the expanded configuration, may for example range from 5 mm to 30 mm).

The patch, when affixed or fastened to the membrane, may be adapted or configured to withstand, e.g., until birth in case of fetoscopy, a certain range of shear forces and pressure that may be imparted in a direction normal to the surface of the patch. For example, amniotic fluid pressure range that may act or be imparted normal to the patch's surface may range from 9 mmHg at gestational stage of week 10 to 5 mmHg at 30 weeks. In TTTS, the amniotic pressure that the patch has to withstand until birth can also go up to 20 mmHg.

The figures illustrate generally, by way of example, but not by way of limitation, various embodiments of the devices and/or methods discussed in the present document.

For simplicity and clarity of illustration, elements shown in the figures have not necessarily been drawn to scale. For example, the dimensions of some of the elements may be exaggerated relative to other elements for clarity of presentation. The number of elements shown in the Figures should by no means be construed as limiting and is for illustrative purposes only. Furthermore, reference numerals may be repeated among the figures to indicate corresponding or analogous elements. References to previously presented elements are implied without necessarily further citing the drawing or description in which they appear. The figures are listed below.

FIG. 1 is a schematic cross-sectional view of a portion of a ruptured amniotic layer sealed with a sealant, according to some embodiments;

FIG. 2A is a schematic assembly illustration of a membrane closure device, according to some embodiments;

FIG. 2B is a schematic illustration of a patch in a folded configuration located at the distal end of the membrane closure device, according to some embodiments;

FIG. 2C is a schematic disassembly illustration of the membrane closure device, according to some embodiments;

FIG. 2D is a schematic illustration of the patch in an expanded configuration after delivery through the distal end of the membrane closure device, according to some embodiments;

FIG. 2E is a schematic illustration of a support structure of a patch, according to an embodiment;

FIG. 2F is a schematic illustration a support structure of a patch, according to an alternative embodiment;

FIG. 2G is a schematic illustration of example measures of a petal or lobe of the support structure of FIG. 2F, according to some embodiments;

FIG. 2H is a schematic illustration of a support structure, according to some other embodiments;

FIGs. 3A and 3B are schematic illustration showing a method of using the membrane closure device, according to some embodiments;

**FIG. 3C** is a schematic illustration of the patch in an expanded configuration, according to some embodiments;

**FIG. 4A** is a schematic illustration of a fastener delivery mechanism of the membrane closure device, according to some other embodiments;

**FIG. 4B** is a schematic illustration of a distal end of the fastener delivery mechanism of **FIG. 4A;**

**FIG. 5** is a schematic illustration of a mixing chamber of the fastener delivery mechanism, according to some embodiments;

**FIGs. 6A** is a schematic illustration showing the patch in a retained state, according to some embodiments;

**FIG. 6B** is a schematic illustration showing the patch in a released state, according to some embodiments;

**FIG. 7** is a flowchart diagram of a method for closing a perforated membrane, according to some embodiments;

**FIGs. 8A** to **8D** show images of an example implementation of a membrane closure device;

**FIGs. 9A** to **9F** show images of another example implementation of a membrane closure device;

**FIGs. 10A** to **10D** show images of an example in-vitro application using the membrane closure device shown in **FIGs. 8A** to **8D;**

**FIGs. 11A** and **11B** show top and side view photograph of an umbrella-shaped receptor without (left) and with (right) Degrapol® membrane coverage, as used in the experiments;

**FIG. 11C** shows the applicator prototype (also: membrane closure device prototype) in its expanded configuration, as used in the experiments;

**FIG. 12A** shows a side view image of an ex vivo fetal membrane model clamped in an aluminum cylinder;

**FIG. 12B** shows a top view image of the tissue clamped in the aluminum cylinder;

**FIG. 12C** shows a bottom view of the tissue clamped in the aluminum cylinder;

**FIG. 13A** shows an image of an umbrella-shaped receptor with Degrapol® membrane on porcine muscle (soft surface) before pulling test;

**FIG. 13B** shows an image of the umbrella-shaped receptor during the pulling test;

**FIG. 13C** shows histograms of the Forces needed to flatten the receptor;

**FIG. 14A** shows histogram of adhesion strengths of umbrellas glued to fetal membranes;

**FIG. 14B** shows an image of an umbrella glued to a fetal membrane defect model with Mussel glue;

**FIG. 14C** shows an image of an umbrella glued to a fetal membrane defect model with Fibrin glue;

**FIG. 14D** shows an image of an umbrella glued to a fetal membrane defect model using Histoacryl®; and

**FIGs. 15A** to **15D** shows images that were recorded by an endoscopic camera of pulling an umbrella against a fetal membrane, followed by glue injection, release of the umbrella and removal of the applicator as well as of the catheter.

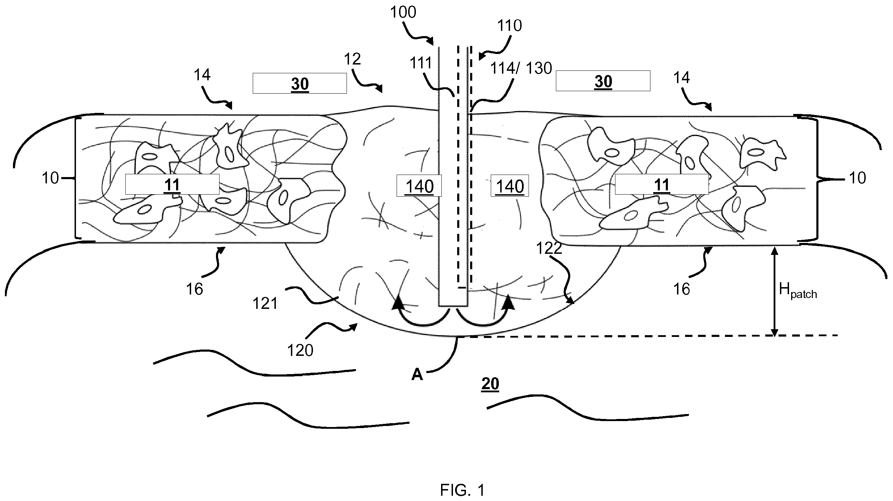

Referring to **FIG. 1,** a membrane **10** consisting of membrane tissue **11** is shown to enclose an amniotic cavity **20.** Membrane **10** prevents fluid contained in the amniotic cavity to leak out to the membrane's surroundings **30.** Membrane **10** has a perforation site **12** extending from a proximal membrane surface **14** to an inner membrane surface **16** of membrane tissue **11.** Perforation site **12** can be sealed in a fluid-impermeable manner by employing a membrane closure device **100,** e.g., as outlined herein below. The term "fluid" as used herein may encompass liquid, gas and/or air. In some embodiments, a patch **120** and/or a fastener **140** may be permeable to gas and/or air, but liquid-impermeable. Optionally, patch **120** and/or fastener **140** may be semi-impermeable, e.g., permeable to allow fluid to permeate from surroundings **30** into amniotic cavity **20** but impermeable for fluid to flow from amniotic cavity **20** into surroundings **30.** Optionally, patch **120** and/or fastener **140** may be semi-impermeable to liquid and permeable to gas and/or air. Optionally, patch **120** and/or fastener **140** may be semi-impermeable to gas and/or air. IN some embodiments, a plurality of patches may be deployable to the same perforation site in a given procedure by membrane closure device **100,** without requiring removal thereof. Optionally, membrane closure device **100** may be operable to contain and sequentially deploy a plurality of patches.

Membrane closure device **100** comprises a patch delivery mechanism **110** that comprises a tube-shaped body **111** for slidably guiding a patch **120** to perforation site **12** and, further, a fastener delivery mechanism **130** for delivering a fastener **140** to perforation site **12**. In some embodiments, lubricants (e.g., oil) may be employed for facilitating the sliding of a patch **120** along the patch delivery passageway.

As exemplified in **FIG. 1,** patch **120** and fastener **140** may be employed for sealing perforation site **12**. For example, patch **120** may be arranged in an expanded configuration (e.g., expanding latitudinally) onto an inner surface **16** of membrane **10** to cover perforation site **12,** and fixed in position by a fastener **140** to seal the perforation. Otherwise stated, fastener **140** may be employed to fixedly attach or secure patch **120** in expanded configuration onto a surface of membrane **10** to seal perforation site **12**. Accordingly, fastener **140** may be operative to sealantly secure patch **120** on membrane **10** to seal perforation site **12**. Generally, one or more patches may be sealantly secured onto outer surface **14** and/or inner membrane surface **16** of membrane **10**. For example, a first patch may be sealantly secured onto inner membrane surface **16** and a second patch may be sealantly secured to outer membrane surface **14**. However, merely to simplify the discussion herein, without be construed limiting, examples and/or embodiments disclosed in conjunction with the accompanying figures refer to sealantly securing patch **120** to inner membrane surface **16.**

In an embodiment, patch **120** comprises a closure sheet **121** that includes, for example, a self-growing sponge, native tissue, living tissue, Polytetrafluorethylen (PTFE), expanded Polytetrafluorethylen (ePTFE) manufactured, e.g., by employing electro-spinning; silicone-based membrane. Such silicone-based membrane may for example be coupled to structures consisting of, for example, synthetic polymer material; natural polymer material, metal; and/or any other suitable matter, or composition of matter. The silicone-based membranes may be treated, e.g., using dip-coating to produce cell-instructive membranes.

Fastener **140** may comprise, for example, native tissue, a glue and/or sealant such as, for example, a biocompatible adhesive and/or a biocompatible sealant. Exemplarily, glue that may be employed can include Mussel glue, agarose glue, fibrin glue, platelet rich plasma, thrombin-containing commercial preparations, collagen, collagen slurry, gelatine sponges, matrigel and/or any other suitable matter.

Optionally, the glue and/or sealant may have a growth factor (e.g., activation with soluble epidermal growth factor (EGF) and/or insulin-link growth factor-1 (ILGF-1); and/or comprise a cell instructive scaffold like, for example, a decellularized amniotic membrane. Optionally, the cell instructive scaffold may be glued (e.g., with fibrin), and for example loaded with mixed, soluble TGF-beta or FGF-beta.

In some embodiments, fastener **140** comprises a weld produced, for example, by a laser source.

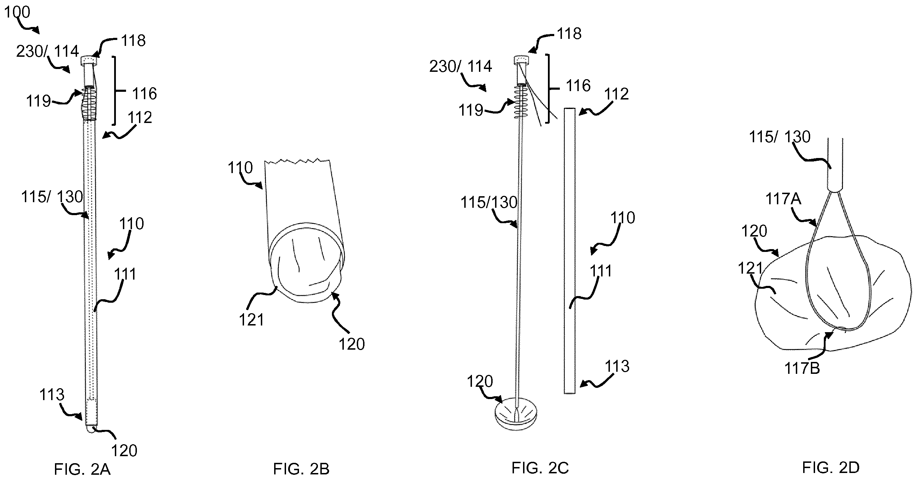

Additional reference is made to **FIGs. 2A** and **2B.** In an embodiment, patch delivery mechanism **110** comprises a tube-shaped body **111** having a proximal end **112** and a distal end **113** and defining internal to the body a patch delivery passageway. Patch delivery mechanism **110** may comprise a patch propelling device **114** that is adapted or configured to be employable by the user of membrane closure device **100** for pushing patch **120** through the patch delivery passageway from its proximal **112** to its distal end **113.**

In an embodiment, patch propelling device **114** may comprise an elongate body **115** that can be introduced into tube-shaped body **111** and which is long enough to protrude out of the distal end of tube-shaped body **111**. In an embodiment, patch propelling device **114** further comprises an actuation mechanism **116** comprising, e.g., a push button **118** and an energy storage device **119** (e.g., a mechanical energy storage device such as a compression spring) for retaining elongate body **115,** and hence patch **120,** by default in a retracted position so that patch is retained inside tube-shaped body **111.** Energy storage device **119** is arranged and configured or adapted to retain push button **118** and hence, elongate body **115** and patch **120** thereto, by default, in the retracted position. For example, energy storage device **119** may be arranged between proximal shoulders of tube-shaped body **111** and shoulders of push button **118.** Clearly, patch propelling device **114** may employ additional or alternative mechanisms for retaining patch **120** by default in the retracted position and for actuating delivery of patch **120** to perforation site **12**. Patch propelling device **114** may comprise, for example, electronic, magnetic, pneumatic, hydraulic and/or any other suitable mechanism.

When the user operably engages with the handle mechanism (e.g., manually presses onto the push button), additional energy is stored by the energy storage device, elongate body **115** advances and pushes patch **120** out of tube-shaped body **111** so that patch **120** protrudes out of the tube-shaped body's distal end **113**. While being pushed through tube-shaped body **111,** patch **120** is in a narrow configuration. Patch **120** in the narrow configuration is schematically and exemplarily shown in **FIGs. 2A** and **2B** in the patch-delivery passageway of tube-shaped body **111,** at its distal end **113.** Once patch **120** is pushed out and no longer confined within the patch delivery passageway running along tube-shaped body **111,** the patch may assume the expanded configuration. When the user then releases the pressure applied onto the push button, added energy is released causing push button to retract into its initial, default position. Clearly, patch delivery mechanism may employ additional and/or alternative handle mechanisms for manually, automatically or semi-automatically propelling patch **120** including, for example, rotary knobs, levers, and/or handles.

Additional reference is made to **FIGs. 2C** and **2D,** showing patch **120** in the expanded configuration. For instance, **FIG. 2C** shows patch **120** in the expanded configuration before being pushed into tube-shaped body **111,** and **FIG. 2D** shows a more detailed view of patch **120** shown in **FIG. 2C.** As schematically shown in **FIG. 2D,** membrane closure device **100** comprises, in an embodiment, a patch manipulation arrangement, e.g., for pulling patch **120** against inner membrane surface **16** and for preventing patch **120** from falling into amniotic cavity **20,** e.g., due to gravitation, after exiting distal end **113.** Such patch manipulation arrangement may for example comprise a patch retention element **117A** which may comprise a handhold **335** comprising a rod that runs within tube-shaped body **111.** The rod of handhold **335** may protrude out of proximal slit **333** so that it can be grasped by the user. A distal end of the handhold's rod may be coupled with patch retention element **117A.** Patch retention element **117A** may comprise a loop to form-fittingly engage with a wire loop **117B** that is affixed to closure sheet **121.** Optionally, patch retention element **117A** may be suture or wire that can be used for suturing of patch **120** to membrane **10.** In some embodiments, membrane closure device **100** may comprise a patch uncoupling mechanism (not shown) comprising, for example, a cutting element (not shown), for cutting wires **117A** and/or **117B** open, allowing both patch delivery mechanism **110** and fastener delivery mechanism **130** be removed from perforation site **12.**

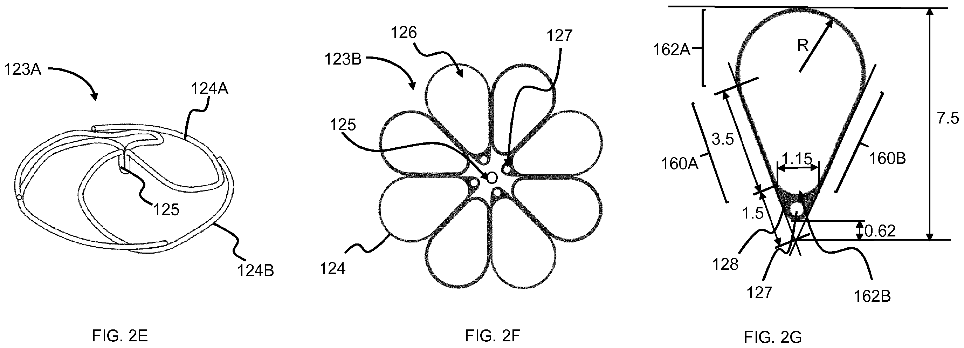

Additional reference is made to **FIGs. 2E** and **2F.** Expandable patch **120** (which may be self-expandable) further comprises a support structure **123. FIGs. 2E** and **2F** schematically show different embodiments of such support structure **123,** respectively referenced by alphanumeric designations **"123A"** and **"123B".**

Generally, support structure **123** is adapted or configured to support closure sheet **121.** Patch 120 may be an expandable (e.g., umbrella-like) device that can be modified from a narrow to an expanded configuration. Optionally, support structure **123** is adapted or configured to permit patch **120** to be actuated to automatically expand from a narrow configuration to an expanded configuration. Expandable patch **120** may assume the expanded configuration after being propelled (e.g., pushed and/or pulled) through tube-shaped body **111** in the narrow configuration and after exiting distal end **113** for closing perforation site **12**

In some embodiments, support structure **123** stores more mechanical energy in the folded or narrow configuration than in the expanded configuration. Accordingly, once support structure **123** is no longer confined within the patch delivery passageway running along tube-shaped body **111,** support structure **123** may attain the expanded configuration.

In some embodiments, support structure **123** may have to be actuated to obtain the expanded configuration. For instance, support structure **123** may be made of a shape memory material (SMA) such as nitinol, and can have a low temperature martensite state, with a martensite transition temperature below body temperature, and a comparatively high temperature austenite state at or above body temperature. Below body temperature, support structure **123** may be in its martensitic state, which is relatively soft, plastic, and easy to shape. In its austenite state, support structure **123** may assume a memorized harder material state in which support structure **123** is more difficult to deform than in the martensitic state. In some embodiments, in order for patch **120** to automatically obtain the desired shape in the expanded configuration, the materials employed for producing support structure **123** may be subjected to temperature-based shape-setting. Heating of closure sheet **121** to above body temperature where it may assume its austenite state with its memorized harder material state may in some embodiments be employed including, for example, electric charge/current flowing via a wire/electric-circuit (not shown) (possibly made of copper) or by other means such as laser light propagating via tube-shaped body **111.**

According to some embodiment, support structure **123,** when in the expanded configuration, may be resistant to bending forces of, for example, at least 1 Newton, 2 Newton, 5 Newton, or at least 10 Newton.

During the transition from the narrow to the expanded configuration, the support structure **123** urges closure sheet **121** to unfold.

In an embodiment, closure sheet **121** may be affixed to support structure **123.** Optionally, support structure **123** may be embedded (partially or fully) in closure sheet **121,** glued to closure sheet **121,** and/or otherwise affixed to closure sheet **121.** Optionally, in the expanded configuration, closure sheet **121** may be stretchably coupled to support structure **123.**

In an embodiment, support structure **123** comprises struts that may for example be made of wires or coils.

As schematically shown in **FIG. 2E,** support structure **123A** may for example comprise at least two wires **124A** and **124B** that assume, in the expanded configuration, an S-shaped form. In the expanded configuration, the at least two S-shaped wires **124A** and **124B** may be rotated relative to each other at their midpoints **125** such that the arcuate portions of the S-shaped wires complement a circular-like strut portion running along the outer edge of closure sheet **121.** Optionally, a support structure **123** may comprise wires that assume in the expanded configuration, T-shaped, U-shaped, C-shaped, longitudinal wires and/or any other desired form.

As schematically shown in **FIG. 2F,** support structure **123B** may comprise wires delineating in the expanded configuration the shape of a plurality of petals or lobes **126.**

Generally, a petal may assume, in the extended configuration, any polygonal shape and therefore have curved and/or straight vertices and/or edges. For example, a petal **126** can have a triangular or substantially triangular shape.

In an embodiment, a wire of support structure **123B** may delineate a curve resembling the shape of petalled flower or have a "Shamrock-like" shape. The curve delineated by support structure **123B** may exemplarily be termed "rhodonea" or "rose curve" and may be constructed using, for example, one of the following polar equations (1) or (2):

$$r=a\ sin(n\vartheta) \tag{1}$$

or

$$r=a\ cos(n\vartheta) \tag{2}$$

As shown exemplarily in **FIG. 2F,** $n=4$, resulting in that the "rose" is 8-petalled. It should be noted that additional or alternative approaches or mathematical formulas can be employed to arrive at a support structure **123B** delineating, e.g., a petalled contour or curve as exemplarily shown in **FIG. 2F,** and that the number of petals or lobes shown in **FIG. 2F** should by no means be construed in a limiting manner. Optionally, a petal or lobe-shaped geometry having the form of a petal **126** may be (virtually) rotated around midpoint **125** for constructing a petalled flower structure.

Further reference is made to **FIG. 2G.** A lobe **126,** which may be made of a wire, may have the measures (in millimeters) as indicated, for example, in **FIG. 2G.**

Each lobe **126** may comprise two radial portions **160A** and **160B** virtually originating from midpoint **125** and terminating in a distal convex curve **132A** having a radius **R.** Optionally, a lobe **126** may comprise a convex curve **132B** that is proximal to midpoint **125.** Optionally, two neighboring lobes **126** may be coupled with each other at adjacent radial portions **160.** Optionally, adjacent radiation portions **160** of two neighboring lobes **126** may abut against each other, yet be detached from one another.

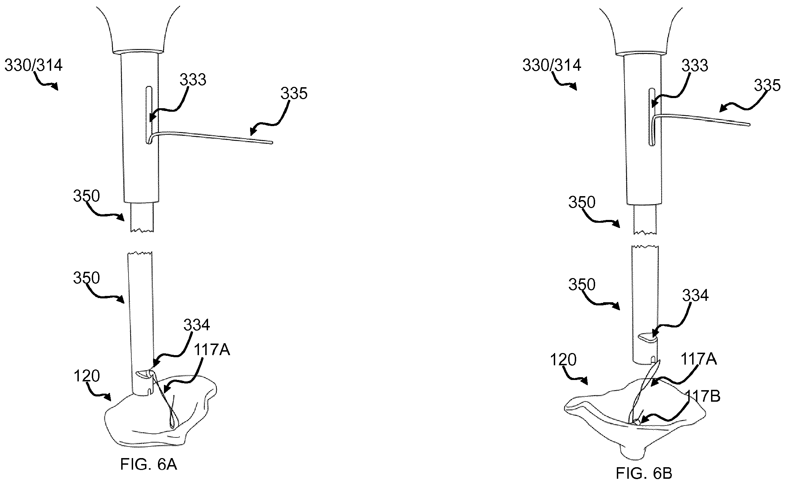

In an embodiment, at least one lobe **126** of support structure **123B** may comprise coupling elements **127,** e.g., for coupling support structure **123B** with patch retention element **117A,** which is exemplified and schematically illustrated in **FIGs. 6A** and **6B.** Optionally, coupling element **127** may be located proximal to geometric midpoint **125** of support structure **123B.** Optionally, lobe **126** may comprise a base **128** that is proximal to midpoint **125.** Optionally, base **128** may comprise coupling element **127.** Optionally, coupling elements **127** may be located distal to geometric midpoint **125** of a support structure, e.g., on the outer rim thereof.

Optionally, two coupling elements **127** of respective lobes **126** may be located opposite each other. Optionally, a coupling element **127** may comprise an eyelet, a hook and/or any other suitable configuration to enable the coupling of support structure **123B,** and thus of patch **120,** with elongate body **115** of patch propelling device **114.**

Optionally, two lobes **126** that are located opposite each other may each include a coupling element **127.** In embodiments where the number of lobes **126** is even, only every second lobes **126** may comprise a coupling element **127.** In some embodiments, lobes **126** may have a substantially symmetrical geometry, e.g., as shown in **FIGs. 2F** and **2G.** In some other embodiments, lobes **126** may have a non-symmetrical geometry (not shown).

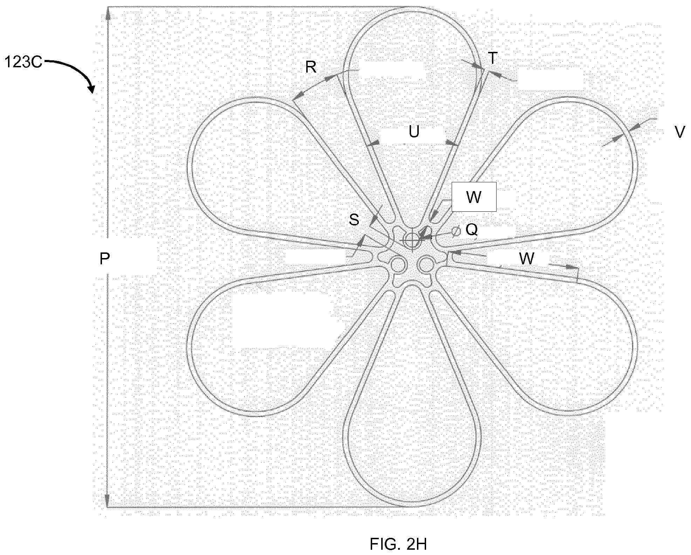

Further referring to **FIG. 2H,** a support structure **123C** may have a similar configuration as support structure **123B** shown in **FIG. 2F,** with the optional difference that at least two or each two neighboring lobes **126** are spaced apart from each other by an angular degree, herein designated by alphanumeric reference "R". More specifically, two juxtaposing radial portions of two neighboring lobes may define an angle of the magnitude **R.** Said angle **R** may for example range from about 5 degrees to about 25 degrees. Optionally, the angle **R** may for example be about 7.5 degrees or about 15 degrees. For example, the lobes of a support structure having six lobes may for example be spaced apart from each other by, e.g., 15 degrees. In another example, the lobes of a support structure having 12 lobes may for example be spaced apart from each other by, e.g., 7.5 degrees. Alternative numbers of lobes and/or angular distances than those exemplified herein may be employed for implementing a support structure.

Optionally, two radial portions of the same lobe of a patch may radially extend from a centerpoint to define an angle **U** ranging, for example, from about 22 to 45 degrees. Optionally, the struts may have a thickness **T** and **V** ranging, for example, from about 100 $\mu$m to about 300 $\mu$m. Optionally, a strut defining a lobe may have varying thickness. Optionally, the struts may have a thickness of about 200 $\mu$m. Optionally, the struts may have a thickness of about 150 $\mu$m. Optionally, a distance **S** between two coupling elements **127** may range, for example, from about 0.1 mm to about 0.3 mm. Optionally, distance **S** may have a magnitude of about 0.117 mm or of about 0.383 mm. Optionally, coupling elements **127** may have a diameter **Q** ranging, for example, from about 0.4 to about 0.5. Optionally, coupling elements **127** may have a diameter **Q** of about 0.556 mm. Optionally, a length **W** of a radial portion may range, for example, from about 3 to about 6 mm. Optionally, a radial portion may have a length **W** of about 3.5 mm or of about 5.282 mm. In some embodiments, the radial portion may have a length **W** which is suitable to accommodate a patch having in the expanded configuration a diameter of, for example, up to 30 mm. For example, the radial portion may have a length **W** of, e.g., at least 12 mm, or at least 15 mm.

In some embodiments, support structure **123** may be manufactured, for example, from a sheet of shape memory alloy (e.g., Nitinol) by employing laser-cutting technologies, and/or by welding and/or braiding of wires. Support structure **123** may be, in some embodiments, basket-like shaped.

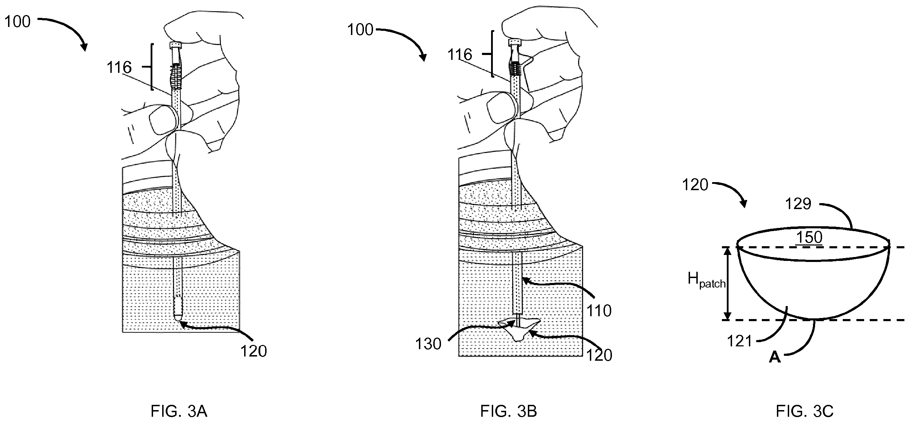

Additional reference is made to **FIGs. 3A** and **3B,** which schematically show a method of using the membrane closure device, according to some embodiments. More specifically, an example of in-vitro deployment of patch **120** by the user is schematically illustrated. **FIG. 3A** shows patch **120** in tube-shaped body **111** in the folded configuration, and **FIG. 3B** shows patch **120** in the expanded configuration.

Further reference is made to **FIG. 3C.** According to some embodiments, patch **120** may form, in the expanded configuration, a concave or an approximately concave surface **122** of height ($H_{patch}$), which may for example be defined as the distance between the rim **129** and the apex **A** formed by closure sheet **121** (cf. **FIG. 1** and **2D**).

The patch's concave surface **122** may facilitate the gathering or holding of glue and/or sealant **140** by closure sheet **121,** as is shown schematically in **FIG. 1,** and prevent the glue and/or sealant **140** to spill into amniotic cavity **20.**

When expanded patch **120** is in an operable position, concave surface **122** may face inner membrane surface **16,** thereby forming a retention cavity **150** that is adapted or configured to receive composition of matter (e.g., glue **140**) that may assume or that has adhesive characteristics, so that patch **120** can adhesively couple with inner membrane surface **16,** e.g., due to polymerization of the composition of matter.

In the expanded configuration, patch **120** may for example assume a height ranging from about 1.5 mm to about 5 mm. Optionally, patch **120** may assume in the expanded configuration a height of about 3 mm.

Optionally, glue **140** may assume adhesive properties only after being delivered to retention cavity **150.** Optionally, glue **140** may already have adhesive properties prior to being delivered to retention cavity **150.** The adhesive strength that may be imparted by glue **140** when patch **120** is adhesively coupled to membrane **10** may range, for example, from about 0.5 Newton to about 1.5 Newton, or from to about 0.5 Newton to about 5 Newton.

According to the invention, the volume of a cavity within fastener-delivery mechanism **130** for receiving glue **140** corresponds or is smaller than the volume of retention cavity **150** (**FIG. 3C**). Correspondingly, fastener-delivery mechanism **130** is configured or adapted so that the amount of glue **140** that can be delivered thereby into retention cavity **150** during one procedure does not exceed the amount of adhesive that can be received by the retention cavity. In other words, membrane closure device **100** is operable to ensure that there is, for a given procedure, a dead volume in retention cavity **150,** i.e., a certain volume of retention cavity **150** remains empty of glue **140.** Thusly configured, the delivery of excess adhesive and, therefore, spilling thereof into amniotic cavity **20** may be prevented. In some embodiments, the difference between in a glue volume that can be delivered by fastener-delivery mechanism **130** and a glue volume that can be received by retention cavity **150** may be, for example, at least about 100 μl, at least about 200 μl, at least about 300 μl, at least about 400 μl, or at least about 500 μl.

Optionally, patch **120** may have to be pulled or held against inner membrane surface **16** circumferencing perforation site **12** for 5 to 180 seconds, to ensure that the patch is sealantly secured to membrane **10.** After patch **120** is secured so that perforation site **12** is sealantly lidded, patch retention element **117A** may be uncoupled (e.g., cut or released) by operably employing the patch uncoupling mechanism (not shown). Patch retention element **117A** and wire **117B** can be biocompatible and, optionally, biodegradable and/or bioabsorbable.

As already briefly outlined herein, membrane closure device **100** comprises a fastener delivery mechanism **130** for delivering a fastener **140** to perforation site **12.** The fastener delivery mechanism may be manually, automatically or semi-automatically operable.

In some embodiments, a fastener delivery mechanism may be a device that is separate and independently operable from a patch propelling device. In some other embodiments, a fastener delivery mechanism may be integral to a patch propelling device. Although the discussion that follows with respect to the accompanying figures refers to embodiments where a fastener delivery mechanism is integral to a patch propelling device, this should by no means be construed limiting.

According to some embodiments, fastener delivery mechanism **130** is adapted or configured to enable controllably delivering glue **140,** e.g., once patch **120** is deployed at perforation site **12** and, optionally, after it assumed the expanded configuration, for securing patch **120** with the delivered glue **140** to membrane **10.** For example, fastener delivery mechanism **130** may comprise a glue injection mechanism (not shown). For example, glue injection mechanism may comprise a cavity defining internal to elongate body **115** a fastener-delivery passageway that is adapted or configured to receive glue **140.** In an embodiment, the glue injection mechanism may further comprise a plunger (not shown) that is provided on the front end thereof with a piston that is slidably movable within the cavities of elongate body **115** for pressing glue **140** out of the fastener-delivery passageway.

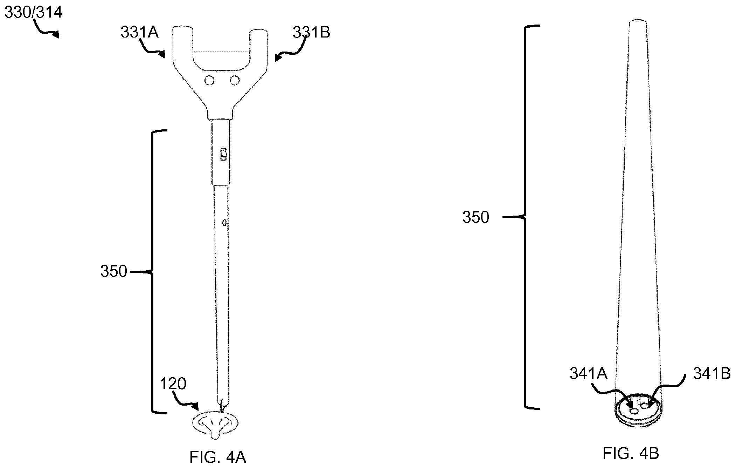

Additional reference is made to **FIGs. 4A and 4B.** In some embodiments, a fastener-delivery mechanism **330** may have a Y-shaped or bifurcated configuration and may comprise at its bifurcated distal end portions a plurality of barrels (e.g., barrels **331A** and **331B**) of a glue injection mechanism (not shown). The plurality of barrels may converge to a shared elongate body portion **350** where they are in separate fluid communication with a plurality of fastener-delivery passageways (e.g., passageways **341A** and **341B**) running along elongate body portion **350** for the separate delivery and, optionally, mixing, of a corresponding plurality of glue compositions of matters (e.g., two glue components). The glue injection mechanism can for example comprise, analogous to the embodiments discussed in **FIGs. 2A** and **2B,** a plurality of plungers (not shown) that are each provided on the front end thereof with a piston (not shown). The piston (not shown) can be slidably movable within the cavities of elongate body of barrels **331A** and **331B** for pressing glue **140** out of the fastener-delivery passageways.

In some embodiments, fastener delivery mechanism **330** may also function as a patch propelling device **314.** Patch propelling device **314** may for example be slidably insurable into tube-shaped body **111** for pushing patch **120** outside the distal end of tube-shaped body **111.**

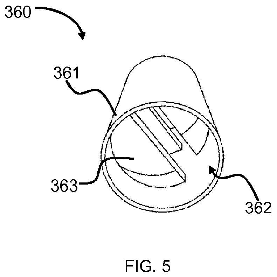

Further reference is made to **FIG. 5.** In some embodiments, fastener delivery mechanism **330** may comprise a mixer **360** operative to mix two or more adhesive-components with each other for producing an adhesive. In some embodiments, elongate body portion 350 may terminate in mixer **360.** Optionally, mixer **360** may be implemented as a static or dynamic mixer.

Mixer **360** may for example comprise a mixing body **361** defining therein a mixing cavity **362.** Fins **363** may protrude inwardly into mixing cavity **362** for the mixing of different adhesive matter of composition. In an embodiment, fins **363** are alternatingly arranged opposite each other along the longitudinal axis of mixing body **361.** In an embodiment, fins **363** are arranged in a screw-like progression along the tube-shaped mixing body.

Additional reference is made to **FIGs. 6A** and **6B.** As already briefly outlined herein, the patch manipulation arrangement of membrane closure device **100** may for example comprise a patch retention element **117A** that runs within tube-shaped body **111** and terminates at its distal end that is releasably coupled with patch **120.** In one embodiment, patch retention element **117A** may be a rod (not shown) that form-fittingly engages with a looped wire or thread **117B** that is affixed to patch **120,** like a latch or plunger. In another embodiment, patch retention element **117A** may comprise a wire or thread that, optionally, runs within elongate body portion **350** and exit therefrom via a distal slit **334** for holding patch **120,** and may further comprise a rod that is releasably coupled with a looped portion of the wire.

Either way, a proximal end of patch retention element **117A** may be coupled to a handhold **335** that protrudes from fastener-delivery passageway, e.g., via a proximal slit **333,** to allow the user to hold it for manipulation of the distal end of patch retention element **117A. FIG. 6A** schematically shows patch **120** in a retained state, e.g., attached to patch retention element **117A. FIG. 6B** schematically shows patch **120** in an uncoupled or released state from patch retention element **117A.** According to the above noted first embodiment, patch **120** may be released by lifting the rod, exemplarily causing the thread to uncouple from elongate body portion **350.** Optionally, the thread may be released from a form-fitting and/or frictional coupling state from the distal end of elongate body portion **350.** According to some embodiments, upon release of the thread, a remainder portion thereof (not shown) may be received within retention cavity **150** and distal end of elongate body portion **350** may be removed in proximal direction from perforation site **12** such that the perforation extending from proximal (also: outer) membrane surface **14** to distal (also: inner) membrane surface **16** (**FIG. 1**) is free of or does not contain any of the following: a portion of the thread, an element of patch **120,** an element of patch delivery mechanism **110,** and an element of fastener delivery mechanism **130.** This allows fetal membrane **10** to slide relative to the uterus (not shown) reducing the risk or without the risk of inflicting damage to either one that would otherwise be present if a stiff element were to extend through the perforation. The perforation itself may optionally be filled essentially only with glue **140.**

In another embodiment, patch **120** may be coupled to the distal part of elongate body portion **350** of fastener delivery mechanism **130.** Optionally, elongate body portion **350** may be slidable from a retracted to an extended position, e.g., by lifting or lowering of handhold **335** which is coupled with patch retention element **117A.** In some embodiments, handhold **335** may be an extension of patch retention element **117A.** Optionally, handhold **335** protrudes out of proximal slit **333.**

Embodiments disclosed herein may be combined with additional wound closure techniques including, for example, cross-linking technologies and/or membrane healing through biological stimulation.

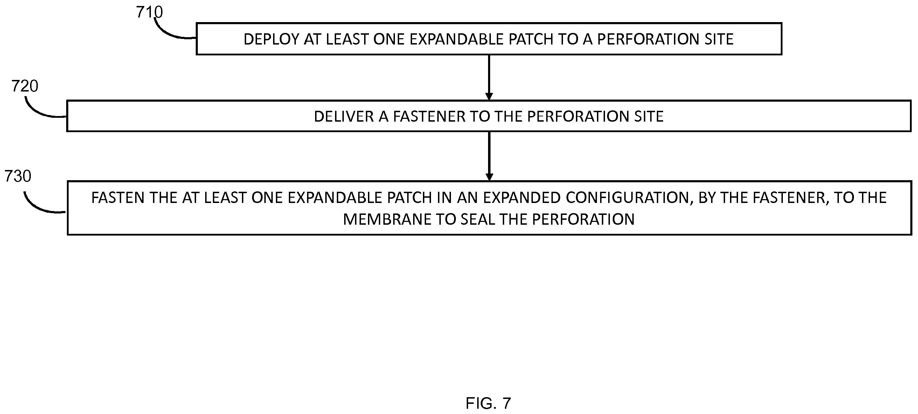

Further reference is now made to **FIG. 7.** As indicated by step **710,** a method for closing a perforated membrane comprises, in an embodiment, deploy at least one expandable patch to a perforation site. Optionally, deploying the at least one expandable patch may include pulling or pushing the patch against the membrane surface to which the patch shall be affixed. For example, referring to **FIG. 1,** patch **120** may be pulled in proximal direction towards the inner surface.

As indicated by step **720,** the method may further include deliver a fastener to the perforation site. As indicated by step **730,** the method may include fastening the at least one expandable patch in an expanded configuration, by the fastener, to the membrane to seal the perforation.

EXAMPLES:

The present disclosure can be better understood by reference to the Example which is offered by way of illustration. The present disclosure is not limited to the Example given herein.

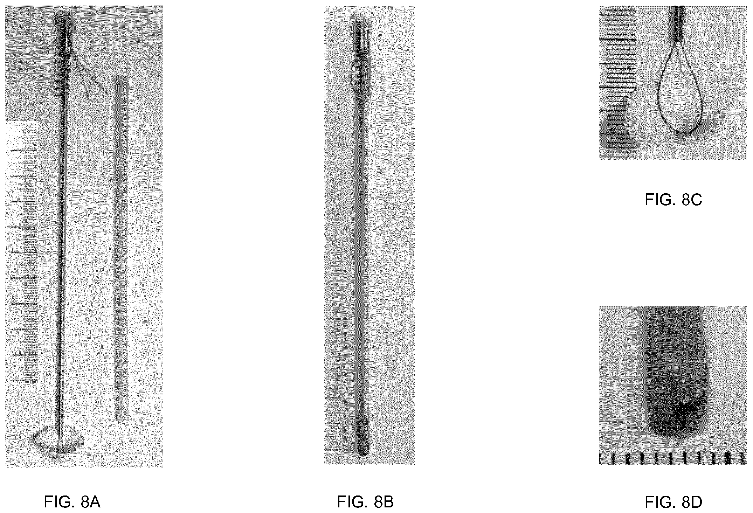

FIGs. 8A to 8D exemplarily show images of an example implementation of a membrane closure device. More specifically, FIG. 8A exemplarily shows an image of the elongate body or pushing cylinder of a patch propelling device responsible of pushing the patch out and retaining it for its positioning onto the uterine wall. FIG. 8B exemplarily shows an image of the membrane closure with the patch loaded inside thereof. FIG. 8C is an image exemplarily showing a more detailed view of the pushing, inner cylinder with the wire retaining the patch when deployed and an inner compartment for glue injection. FIG. 8D exemplarily shows that the transparent appearance of the patch in the folded state is due to the employment of oil that was necessary to load the patch into the tube.

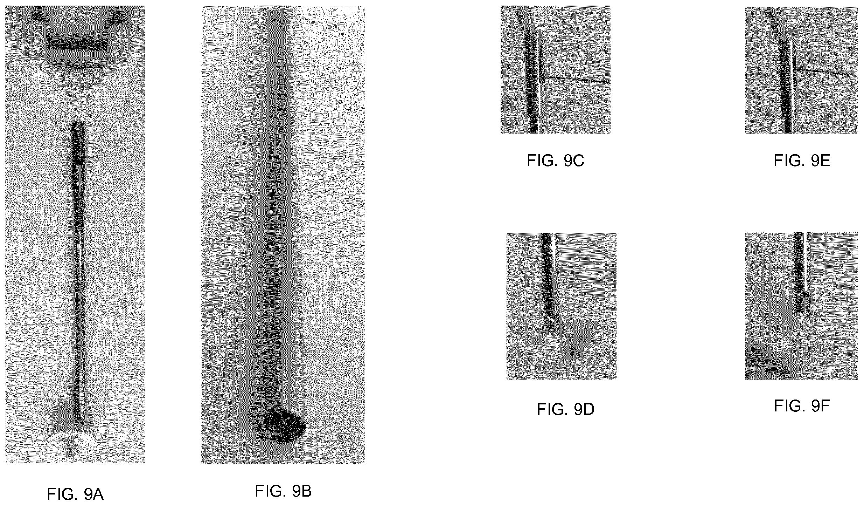

FIGs. 9A to 9F show images of another example implementation of a membrane closure device. More specifically, FIG. 9A shows an image of an example inner cylinder responsible for pushing the patch out and retaining it for its positioning onto the uterine wall. FIG. 9B is an image exemplarily showing an image of two smaller tubes that extend throughout the whole construct of the pushing cylinder to deliver a sealant composed of two components. FIGs. 9C and 9D are image exemplarily showing the membrane closure device with the patch attached to it via a suture wire loop and retained by a wire controlled at the top here in its closed state. FIGs. 9E-9F exemplarily shows this same wire in its open state (patch retention element 117A is uncoupled from wire loop 117B) for releasing the patch.

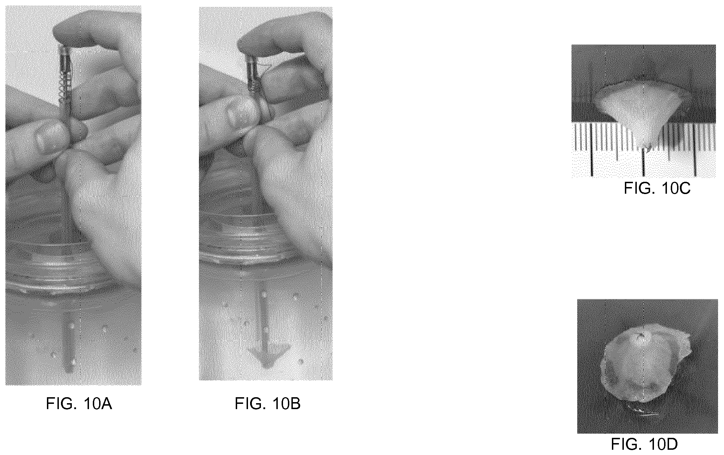

FIGs. 10A and 10B show images of an example in-vitro application of the membrane closure device shown in FIGs. 8A to 8D. More specifically, FIGs. 10A and 10B show the deployment of the patch in a wet container.

FIG. 10C and 10D show different views of the patch affixed to a wet surface with Agarose glue.

The membrane closure devices and components shown in FIGs. 8A to 10D fitted perfectly to the instruments used in clinics for fetoscopy as tested directly on catheters provided by the obstetric department. A metallic mold for strict reproducible receptor fabrication was produced and a series of umbrellas were electrospun. Their compatibility to the membrane closure devices was verified by simple insertion into it and its deployment tested in water. A test of feasibility included injecting fibrin glue through the two-chamber syringe into the concave surface of the patch. The reception capacity was confirmed by this experiment. A nitinol umbrella backbone was manufactured by MeKo™. The nitinol umbrella backbone was embedded into a silicone sheet (the closure sheet). Optionally, the nitinol umbrella backbone could be coated by the electro-spun membrane.

In an additional example, a membrane closure device was employed that comprises an umbrella-shaped receptor (also: expandable patch) and an applicator (also: patch delivery mechanism). The receptor was pushed through a catheter by the applicator and expanded automatically when pushed into the amniotic cavity. The receptor was then positioned and glued against the amnion at the defect site (also: perforation site). The adhesion strength of multiple glues was tested. Additionally, the feasibility and reproducibility of this fetal membrane sealing approach was tested on an ex vivo model. The receptor opened in all tests and its positioning showed no particular difficulties (n = 10). When applied via the minimal invasive procedure, receptors were glued efficiently onto the membrane and all of them completely covered the defect (n = 5). The time needed for the whole procedure was slightly over 3 minutes.

The receptor for the gathering of adhesive materials, ii) the applicator to introduce the receptor (also: patch or umbrella) and iii) the injection system (also: fastener delivery mechanism) to deliver the adhesive materials inside the receptor were assembled in one single device and enabled carrying out the sealing procedure in five short steps. The idea was (1) to insert the receptor through the fetoscopy catheter, (2) deploy it inside the amniotic cavity, (3) pull it back against the inside wall of the fetal membrane and (4) glue the umbrella onto the fetal membrane. (5) The catheter is then removed while the umbrella is left glued to the membrane until it comes out with the baby's birth.

The Applicator shown in is responsible to push the Umbrella inside the 10 French introducer (not shown). The Glue injection mechanism (also: fastener delivery mechanism) is the entry site for the glue, which was delivered to the Umbrella with the assistance of a double chambered tube placed inside the Applicator. A release mechanism of the Umbrella is controlled by one small metallic wire going through the Applicator (Lock). When in the Locked position, the wire traps the suture wire connected to the umbrella. When in the unlocked position, the suture wire is no longer trapped and the umbrella can be unconnected from the Applicator.

Components that were used in the experiments for the preventive sealing of fetal membranes

Umbrella-shaped receptor for the gathering of sealing materials:

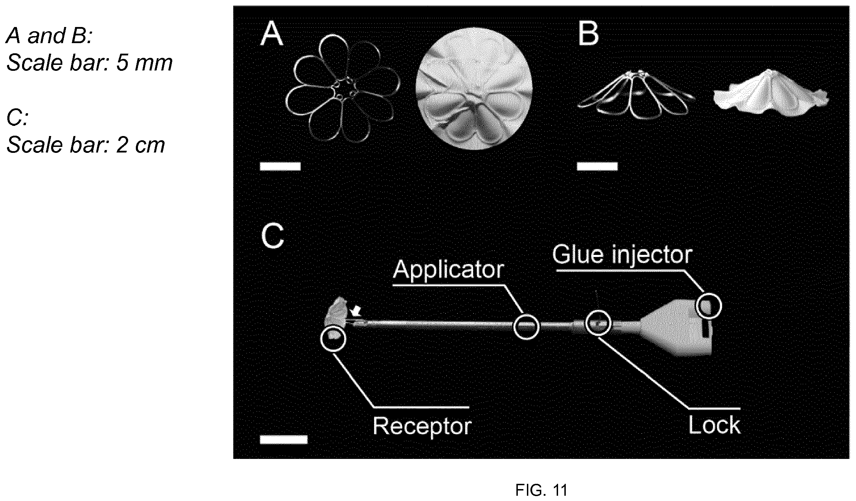

Reference is made to FIGs. 11A-11B. The receptor comprised two elements: a nitinol backbone and a membrane covering that backbone in order to gather the adhesives. The nitinol stripes thickness was set at 200 $\mu$m and the umbrella-shaped height at 3 mm. The receptor was composed of 8 identical oval struts each connected to the neighbouring one, forming a circular shape. The connection of all small part of each oval formed a small circle with a diameter smaller than the 10 French catheter at the proximal part of the umbrella (**FIG. 11A**). They were produced by laser cutting followed by shape setting.

Degrapol® was chosen because of its in-house production availability and its similar stretchability. In addition, it has previously shown a capacity to integrate into rabbit fetal membranes and promote reepithelialisation. (Ochsenbein-Kolble, N., et al. Enhancing sealing of fetal membrane defects using tissue engineered native amniotic scaffolds in the rabbit model. American journal of obstetrics and gynecology 196, 263 e261-267 (2007).

To produce those sheets, 600mg of lyophilized Degrapol® was dissolved in 3.52g of Chloroform and 0.88g of Hexafluoroisopropanol to make 5g of 12% Degrapol® mix. The solution was left at room temperature overnight and 2 ml of this solution was electro-spun at a rate of 1ml per hour on 12 glass slides rotating on the collector. The distance between the syringe needle and the collector was 10cm. The resulting Degrapol® sheets of approximately 150µm thickness were removed from the glass slides by immersing in 30% ethanol. The nitinol backbone was inserted between two Degrapol® sheets that were pressed together to fuse them.

Applicator and injector:

Further reference is made to **FIG. 11C.** A stainless-steel tube with an outer diameter of 2.9mm and inner diameter of 2.7mm was designed and manufactured. This tube slid freely in the 10 French catheter and was tight enough to push the receptor (**Figure 11C,** "Receptor") through the catheter. A double chamber injector (Duplocath 180, Baxter AG, Volketswil, Switzerland) from the Tisseel® glue pack was inserted into the hollow tube from the proximal to the distal end to ensure that the two glue substrates only merge at the distal end of the tube into the receptor. The proximal end consisted of two entry sites for two syringes, each for one component of a two precursors glue.

The receptor had to remain attached to the applicator in order to prevent the loss of it in the amniotic cavity upon deployment and to allow the pulling against the fetal membrane defect. To do so, a thread was attached to the umbrella-shaped receptor and connected it to a locking system at the distal part of the applicator. Upon unlocking of this system by the user, the thread was set free and the receptor was detached from the applicator.

Bioadhesives:

To test the fetal membrane sealing procedure Histoacryl® (B.Braun Surgical AG, Melsungen, Switzerland), fibrin glue (Tisseel®, Baxter AG, Volketswil, Switzerland), and mussel glue were employed (Brubaker, C.E., Kissler, H., Wang, L.J., Kaufman, D.B. & Messersmith, P.B. Biological performance of mussel-inspired adhesive in extrahepatic islet transplantation. Biomaterials 31, 420-427 (2010); Burke, S.A., Ritter-Jones, M., Lee, B.P. & Messersmith, P.B. Thermal gelation and tissue adhesion of biomimetic hydrogels. Biomedical materials 2, 203-210 (2007); Lee, B.P., Dalsin, J.L. & Messersmith, P.B. Synthesis and gelation of DOPA-modified poly(ethylene glycol) hydrogels. Biomacromolecules 3, 1038-1047 (2002); Lee, H., Lee, B.P. & Messersmith, P.B. A reversible wet/dry adhesive inspired by mussels and geckos. Nature 448, 338-341 (2007); Lee, H., Scherer, N.F. & Messersmith, P.B. Single-molecule mechanics of mussel adhesion. Proc Natl Acad Sci U S A 103, 12999-13003 (2006).

Histoacryl® is a cyanoacrylate-based, Federal Drug Administration (FDA) approved one component glue that polymerizes upon contact with hydroxyl ions present in wet environments. Due to its strong gluing ability, it is used for application such as net fixation for inguinal hernia.27 Histoacryl® was directly transferred from its ampoule to a syringe tube before application. Fibrin is another commonly used tissue sealant that showed potentially good binding ability to fetal membranes.21 Fibrin glue is composed of two precursors, Sealer Protein (human plasma) and Thrombin, which are injected simultaneously inside the two-chamber injector tube and polymerize instantaneously when gathered in the receptor. The mechanism of action replicates the formation of blood clot in the vascular system where thrombin activates the coagulation cascade.

Mussel glue is a sealant that readily polymerizes upon mixing of the polymer precursor with its activator and was shown to have very good properties for the sealing fetal membranes under wet conditions.20 As previously described the polymer substrate is dissolved in 2x PBS and mixed in a 1:1 volume ratio to 12mg/ml sodium periodate for a final mussel glue concentration of 150mg/ml.

Human fetal membranes

Human fetal membranes were collected from patients with written consent following the decision from the Ethical Committee of the District of Zürich (study Stv22/2006). Fetal membranes were harvested after caeserian section at term (between 37 and 39 weeks) and frozen at -80°C until usage. All of them were negatively selected for HIV, hepatitis A and B, diabetes mellitus, chlamydia, cytomegaly and streptococcus B.

Mechanical testing

Mechanical stability of the receptor

The mechanical stability of the receptor was assessed by measuring the force needed to completely flatten the receptor. A thread was attached to the center culm of the receptor with its hollow part facing down. The thread was then pulled until the receptor was flattened and the force needed to do so was measured with a dynamometer. Receptors with and without Degrapol® membrane were pulled on solid (Petri dish) and soft surfaces (1.5 cm thick pork muscle).

Adhesion strength provided by bioadhesives to glue the receptor to intact fetal membranes

Intact fetal membranes were flattened on a Petri dish with the amnion facing up. The receptors were placed on top of the membrane followed by the injection of 500μl of glue inside the receptors through a syringe and by 5 minutes of polymerization time. The adhesion strength provided by the glues was measured with a dynamometer (Pesola AG, Schindellegi, Switzerland, ref.DO29/FH27 by pulling the receptors perpendicularly to the membrane surface.

Application and gluing of receptor on punctured fetal membranes

Dry experimental conditions

A fetal membrane was mounted onto a plastic cup with the amnion facing downward to the interior of the cup. A very high bonding elastomeric membrane (3M AG, Rüschlikon, Switzerland) was punched with a 6mm diameter biopsy punch and deposited onto the fetal membrane. A sharp obturator (Anklin AG, Switzerland) was used to punch through the fetal membrane to create the defect. A 10 French catheter (Check-Flo Performer® Introducer, Cook Medical, Bloomington, IN, USA) was introduced in the defect and the receptor was introduced through it into the interior of the cup. The receptor was held for 5 minutes against the fetal membrane following injection of Mussel Glue, Fibrin or Histoacryl®. The membranes were unmounted from the plastic cup and turned upside down. The umbrella was pulled perpendicularly to the membrane surface and the force needed to remove the receptor from the membrane was measured with a dynamometer.

Complete ex vivo fetal membrane defect model

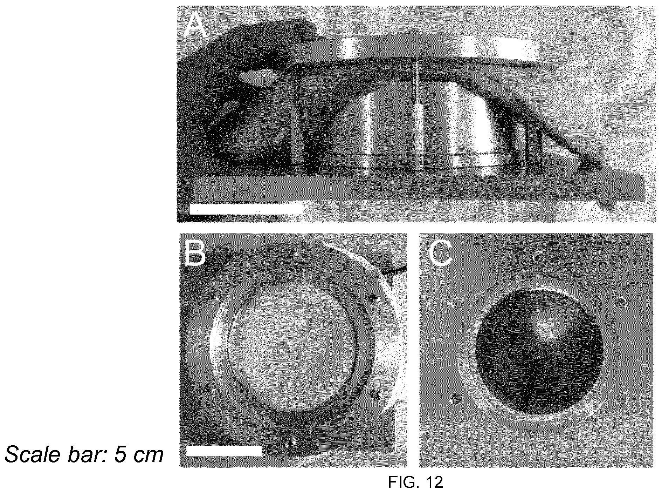

Reference is now made to **FIGs. 12A-12C. FIG. 12A** shows a side view image of an ex vivo fetal membrane mode clamped in an aluminum cylinder; **FIG. 12B** shows a top view image of the tissue clamped in the aluminum cylinder; and **FIG. 12C** shows a bottom view of the tissue clamped in the aluminum cylinder. Porcine skin containing a small muscle layer with a total thickness of 0.5cm was chosen to mimic the upper abdominal wall. A porcine muscle of 1cm thickness and a human fetal membrane were added under the abdominal wall mimic to reproduce the uterus layer. Those three layers were put together, stretched, placed onto a 8cm diameter aluminum cylinder and tightly clamped by tightly screwing a ring on top. The construct has a transparent bottom for visualization purposes and was water tight. Two inlets in the cylinder enabled the cylinder to fill with solutions reproducing the amniotic fluid. Another inlet permitted the insertion of an endoscopic camera in order to have a visual feedback of the interior of the cavity.

A 5 mm cut was made with a scalpel on the skin mimic to facilitate the introduction of a sharp obturator (Anklin AG, Switzerland), which was used to punch through the abdominal and uterine wall mimic as well as through the fetal membrane. A 10 French catheter (Check-Flo Performer® Introducer, Cook Medical, Bloomington, IN, USA) used in fetoscopic interventions was introduced at the same time by mounting it on obturator. The receptor was crimped and inserted in the catheter before pushing it inside the cavity filled with saline solution. The receptor was pulled against the membrane and 500 μl of glue (100 μl corresponding to the dead volume of the double-chamber injection channel plus 400 μl adhesive) was perfused through the injector into the receptor using syringes connected to the injection inlets. Polymerization was allowed for 1 minute while the receptor was held against the fetal membrane in its umbrella-shaped conformation. A video-intubation system (Acutronice Medical Systems AG, Switzerland) enabled the visual control over the placement of the receptor. A total of 4 defects were sealed on one model. After the procedure membranes were released from the cylinder clamp and unmounted from the plastic cup or cylinder devices and turned upside down for further evaluations.

Results:

As the central part of the strategy explained here, the receptor was designed such that it fits through the catheter, deploys into a larger, predefined shape, gathers the adhesive at the site of defect and remains glued to the amnion until delivery. A circular structure was adopted, which structure was made of Nitinol, which enables the crimping into the catheter and spontaneous deployment into the original shape upon exit of the catheter. This metallic backbone was integrated into an elastic Degrapol® membrane to confine the adhesives inside the receptor and limit the exchange with the amniotic fluid. Additionally, to enable the user to feel a resistance before its flattening or even everting, the receptor was given a curvature of 3 mm. The applicator, by which the receptor is pushed through the catheter into the amniotic cavity, was made out of a tube with an outer diameter (3 mm) and a length exceeding the catheter's size by 10 mm. The applicator was shown to slide through the 10 French catheter with a minimal force ($0.2N \pm 0.02N$) and access the inside of the amniotic cavity.

The resistance of receptors with or without Degrapol® was compared against each other to evaluate the contribution of the membrane to the receptor's stability. In addition, an assay was performed on a plastic Petri dish or on a 1.5cm thick muscle layer, which assay was aimed at determining the impact of the support on which the receptor is measured.

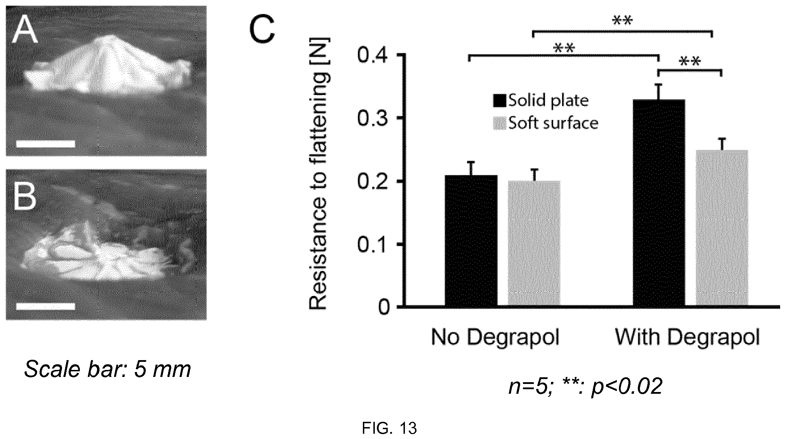

Reference is made to **FIGs. 13A** to **13C. FIG. 13A** shows an image of an umbrella-shaped receptor with Degrapol® membrane on porcine muscle (soft surface) before pulling test. **FIG. 13B** shows an image of the umbrella-shaped receptor during the pulling test. **FIG. 13C** shows histograms of the Forces needed to flatten the receptor.

While the backbone on the stiff and the soft surface deformed at almost identical forces of $0.21 \pm 0.02$ N and $0.2 \pm 0.02$ N, respectively, the resistance to deformation of the full umbrella was significantly increased on both soft and stiff substrates. The force for flattening was significantly higher on the solid substrate ($0.33 \pm 0.02N$) as compared to the soft substrate ($0.25 \pm 0.02N$). This indicates that the receptor needs to be tested with the membrane on a soft tissue to ensure the relevance of the assay for its in vivo application.

### Adhesion strength of the receptor to intact fetal membranes

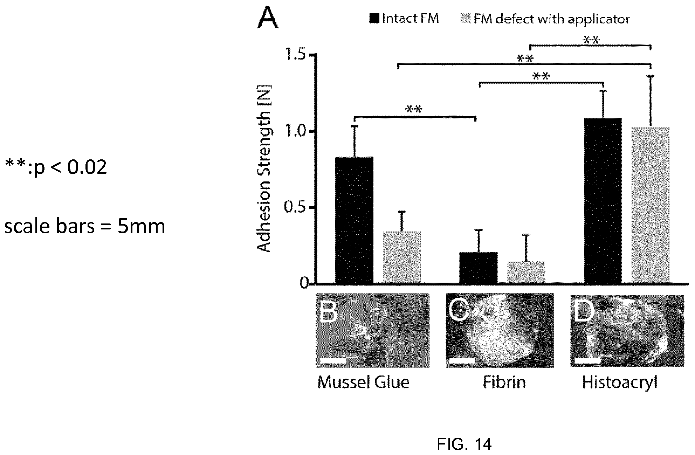

Additional reference is made to **FIGs. 14A** to **14D. FIG. 14A** shows histogram of adhesion strengths of umbrellas glued to fetal membranes. **FIG. 14B** shows an image of an umbrella glued to a fetal membrane defect model with Mussel glue. **FIG. 14C** shows an image of an umbrella glued to a fetal membrane defect model with Fibrin glue. **FIG. 14D** shows an image of an umbrella glued to a fetal membrane defect model using Histoacryl®. The black bars of the histogram illustration in **FIG. 14A** represent the cases where the receptors were directly glued on intact fetal membranes. Grey bars represent the cases when the receptors were applied through the defect and glued on the fetal membrane under dry conditions.

In the first step, we evaluated the potential of three adhesive candidates to glue the Degrapol® coated receptors to the fetal membrane. For this the receptor was deposited on intact fetal membranes and the adhesive was injected in the receptor using a syringe. After 5 minutes of polymerization, the force to detach the receptor from the membrane was measured. While mussel glue reached similar adhesion forces as Histoacryl® ($0.83 \pm 0.2N$ and $1.1 \pm 0.17$ N respectively, n=5), the adhesion of Fibrin with $0.2 \pm 0.14N$ (n=5) was very weak (**FIG. 14A,** black bars). As a drawback, Histoacryl® had the effect of drying out both fetal and the Degrapol® membranes.

### Application and gluing of the receptor on fetal membrane defects under dry experimental conditions

Next, we assessed the ability of the receptor to be pushed through the catheter, to deploy, to be positioned and glued on the fetal membrane. This was done on a model consisting of a punctured fetal membrane and a transparent elastomeric membrane in dry experimental conditions. In all the trials (n=13), the umbrellas deployed to their initial shape, were placed tightly on the fetal membrane and no leakage of the glue was observed upon injection. In addition, all the receptors adhered to the fetal membrane following adhesion and release. However, all the glues showed different values of adhesion strength. Histoacryl® resisted up to $0.96 \pm 0.38N$ (n=3) of pulling force until detachment, compared to $0.35 \pm 0.12N$ (n=5) and $0.17 \pm 0.18N$ (n=5) for Mussel glue and Fibrin respectively (**FIG. 14A,** grey bars).

### Fetal membrane defect sealing procedure on complete ex vivo model

To conclude the proof of feasibility of the approach, a physiologically relevant model was created for the simulation of the catheterization and the sealing of the fetal membrane with our device. An ex vivo model was established to mimic the abdominal, the uterus and fetal membrane layers by the assembly of porcine skin containing a small muscle porcine muscle and a human fetal membrane. The resulting closed cavity was then filled with saline solution, which mimicked the amniotic fluid and provided a more realistic value to the ex vivo model. The tissue mimic had a thickness comparable to the human morphology and besides conferring a physiologically more relevant model also had a higher mechanical stability, which contributes to the resistance strength when pulling the receptor against the membrane. To quantify the mechanical stability, the force needed to pull up the center of the model by 1.5cm was measured. The ex vivo model required 1 N for this displacement, compared to 0.1 N in the model where the fetal membrane was only supported by an elastomeric membrane.

## Defect sealing ex vivo

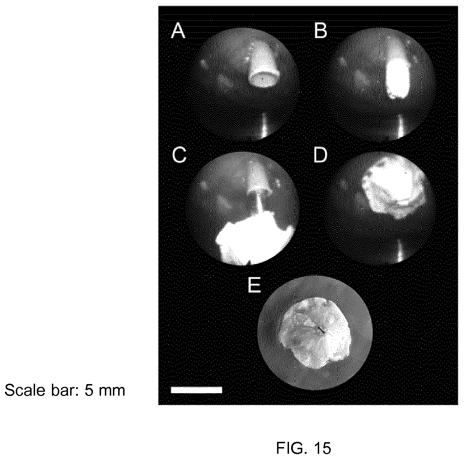

Mussel Glue was taken as the adhesive of choice to be tested in the ex vivo model. A 5 mm incision was made on the skin to facilitate punching through the tissue layers. The 10 French catheter was mounted on the obturator and inserted through the tissue mimic in a 45° angle. The obturator was removed and the catheter left in the defect. The receptor was crimped, inserted in the catheter and pushed with the applicator until deployment inside the cavity. The umbrella was pulled against the fetal membrane, followed by glue injection, release of the umbrella and removal of the applicator as well as the catheter. The procedure was recorded by an endoscopic camera, which steps of the procedure are shown in the images of **FIGs. 15A** to **15E.**

More specifically, **FIGs. 15A** to **15E** illustrate the procedure from inside of the cylinder, reproducing the view from the amniotic cavity, wherein **FIG. 15A** shows accession of the catheter (blue tube) right after punching through the construct to create the defect, in **FIG. 15B** the white element shows the umbrella in its crimped comformation being introduced, **FIG. 15C,** when deployed and **FIG. 15D** when pulled against the fetal membrane. **FIG. 15E** shows an image when the Mussel Glue was absorbed by the Degrapol®, changing its color to red. The figure also shows the umbrella glued onto the fetal membrane after dismantling.

In all trials (n = 5), the receptor opened into its original shape and was properly placed onto the defect. The glue was well confined in the umbrella despite a few observations of minimal leakage. These were mainly due to the slight permeability of the Degrapol® but it never impaired the adhesion of the receptor to the fetal membranes. Indeed, all the receptors held onto the fetal membranes upon release and removal of the applicator. The strength at which they were glued to the membrane was then evaluated the same way as described in the two previous models. On average, 0.13 ± 0.04N were needed to detach the receptor from the fetal membranes.

## Time of procedure

The time needed for each step of the procedure were reported, which can be a potential limitation for the adoption of our method. Those time intervals were summarized in **Table 1.** Device application, which included crimping of the receptor and placement of it onto the fetal membrane, was the longest step of the procedure with an average of 1 minute and 28 ± 43 seconds. Glue injection time had a constant 1 minute polymerization period in all the tests, to which 27 ± 7 seconds were needed to place the syringes on the glue injection inlets and apply the glue. Finally, 13 ± 9 seconds were added to activate the locking system and release the receptor. In summary, the total average time for the whole procedure was 3 minutes and 08 seconds, which represents an insignificant increase of time for fetoscopy.

**Table 1:** Statistics of the fetal membrane sealing procedure on an ex vivo model.

| | Time [min:sec] | Comment | Success rate |
|---|---|---|---|
| *Device application* | 1:28 ± 0:43 | Umbrella deployed and was placed on the defect in every test. | 5/5 (100%) |
| *Glue injection* | 1:27 ± 0:07 | Injection was straight forward. Some leakage through Degrapol® was observed due to the relative permeability of this material, without hindering gluing capacity. | 5/5 (100%) |
| *Umbrella release* | 0:13 ± 0:09 | All the umbrellas remained attached to the membrane upon release. | 5/5 (100%) |
| **Adhesive forces of glued umbrellas [N]** | 0.13 ± 0.04 | | |

## Discussion of the experiments:

The experiments proved the feasibility of our method for the precise application of tissue sealants at the site of fetal membrane defect. To this end, the model that was developed reproduced close to real anatomic conditions to enable the realistic simulation of the procedure ex vivo. As for the method to seal the defect a device was designed and produced that facilitates the precise deposition of bioadhesives at the ruptured site, which is believed to be a significant reason for the limited success of previous attempts to seal the membrane.

The deliberate choice of diverse sealing materials - two-component glues like fibrin or the mussel glue and Histoacryl® - was done to demonstrate the compatibility of our method to materials of different natures and to highlight the relevance of our method for the testing of those injectable candidates.

Unless otherwise stated, the use of the expression "and/or" between the last two members of a list of options for selection indicates that a selection of one or more of the listed options is appropriate and may be made.

In the claims or specification of the present application, unless otherwise stated, adjectives such as "substantially" and "about" modifying a condition or relationship characteristic of a feature or features of an embodiment of the invention, are understood to mean that the condition or characteristic is defined within tolerances that are acceptable for operation of the embodiment for an application for which it is intended.

It should be understood that where the claims or specification refer to "a" or "an" element and/or feature, such reference is not to be construed as there being only one of that element and/or feature.

Terms used in the singular shall also include the plural, except where expressly otherwise stated or where the context otherwise requires.

In the description and claims of the present application, each of the verbs, "comprise" "include" and "have", and conjugates thereof, are used to indicate that the object or objects of the verb are not necessarily a complete listing of components, elements or parts of the subject or subjects of the verb.

The various features and steps discussed above can be mixed and matched by one of ordinary skill in the art in accordance with principles described herein.

Although the disclosure has been provided in the context of certain embodiments and examples, it will be understood by those skilled in the art that the disclosure extends beyond the specifically described embodiments to other alternative embodiments and/or uses and obvious modifications and equivalents thereof. Accordingly, the disclosure is not intended to be limited by the specific disclosures of embodiments herein.

In the description, unless otherwise stated, adjectives such as "substantially" and "about" that modify a condition or relationship characteristic of a feature or features of an embodiment of the invention, are to be understood to mean that the condition or characteristic is defined within tolerances that are acceptable for operation of the embodiment for an application for which it is intended.

It is appreciated that certain features of the invention, which are, for clarity, described in the context of separate embodiments or examples, may also be provided in combination in a single embodiment. Conversely, various features of the invention, which are, for brevity, described in the context of a single embodiment, may also be provided separately or in any suitable subcombination or, as suitable, in any other described embodiment of the invention. Certain features described in the context of various embodiments are not to be considered essential features of those embodiments, unless the embodiment is inoperative without those elements.

It is important to note that methods discussed herein are not limited to those diagrams or to the corresponding descriptions. For example, the methods may include additional or even fewer processes or operations in comparison to what is described in the figures. In addition, embodiments of the method are not necessarily limited to the chronological order as illustrated and described herein.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

Unless otherwise specified, the terms 'about' and/or 'close' with respect to a magnitude or a numerical value may imply to be within an inclusive range of -10% to +10% of the respective magnitude or value.

It is noted that the terms "operable to" can encompass the meaning of the term "adapted or configured to". In other words, a machine for example that is "operable to" perform a task can in some embodiments, embrace a mere capability (e.g., "adapted") to perform the function and, in some other embodiments, a machine that is actually made (e.g., "configured") to perform the function.

As used herein, the phrase "A, B, C, or any combination of the aforesaid" should be interpreted as meaning all of the following: (i) A or B or C or any combination of A, B, and C, (ii) at least one of A, B, and C; and (iii) A, and/or B and/or C. This concept is illustrated for three elements (i.e., A, B, C), but extends to fewer and greater numbers of elements (e.g., A, B, C, D, etc.).

While the invention has been described with respect to a limited number of embodiments, these should not be construed as limitations on the scope of the invention, but rather as exemplifications of some of the embodiments.

**Claims**

1. A membrane closure device (100) for closing a perforation in a membrane (10) having a proximal side and a distal side, the membrane closure device (100) comprising:

   a patch (120) that is selectively expandable from a narrow to an expanded configuration and having in the expanded configuration a concave surface (122) defining a retention cavity (150) for receiving glue (140);
   a patch delivery mechanism (110) for guiding the patch (120) through a perforation of the membrane (10) from a proximal to a distal side thereof;
   a fastener delivery mechanism (130, 330) operable to contain glue (140) and further operable to deliver the glue (140) contained therein from the proximal side to the distal side of the membrane (10) for fastening the patch (120) in the expanded configuration, by the glue (140), to the distal side of the membrane (10) to seal the perforation
   **characterized in that**
   the retention cavity (150) defines a volume that is larger than a volume of glue (140) that can be contained, at any given time, by the fastener delivery mechanism (130, 330).

2. The membrane closure device (100) according to claim 1, comprising at least one tube having a distal and a proximal end for delivering the patch (120) in a folded configuration via the at least one tube from the distal to the proximal end for attaching the patch (120) in an expanded configuration to an inner surface (16) of the membrane (10).

3. The membrane closure device (100) according to any one of the preceding claims, wherein the patch (120) is a self-expandable patch (120).

4. The membrane closure device (100) according to claim 3, wherein the self-expandable patch (120) is an umbrella-like device comprising a support structure (123A, 123B) including struts and a closure sheet (121) affixed to the struts.

5. The membrane closure device (100) according to claim 4, wherein the struts comprise at least two wires (124A, 124B) that assume, in the expanded configuration, an S-shaped form or the form or contour of a petalled flower.

6. The membrane closure device (100) according to claim 5, wherein the struts comprise shape-memory material composed of, for example, Nitinol.

7. The membrane closure device (100) according to any one of the preceding claims, wherein the glue (140) comprises a biocompatible adhesive, a biocompatible sealant, a sealant-inducing material, and/or a healing-inducing material.

8. The membrane closure device (100) according to any one of the preceding claims, wherein the fastener delivery mechanism (130, 330) comprises a mixer (360) that is included in a tube-shaped body (111) of the device for mixing of components to produce an adhesive.

9. The membrane closure device (100) according to claim 8, wherein the mixer (360) comprises a mixing body (361) and fins (363), wherein the fins (363) protrude inwardly into the mixing body (361) for mixing of different adhesive matter of composition.

10. The membrane closure device (100) according to any one of the preceding claims, comprising a patch uncoupling mechanism for releasing the patch (120) from the membrane closure device following fastening of the patch (120) to the inner surface (16) of a perforation site (12) of the membrane (10).

11. The membrane closure device (100) according to any one of the preceding claims, wherein the glue (140) comprises any one of the following: a growth factor and/or a cell instructive scaffold like, for example, a decellularized amniotic membrane.

12. The membrane closure device (100) according to any one of the preceding claims for use in treating a perforation of a fetal membrane, for use in treating a perforation of a vascular membrane, for use in treating a perforation of a cardiovascular membrane and/or for use in treating a tympanic membrane.

**Patentansprüche**

1. Eine Membranverschlussvorrichtung (100) zum Verschließen einer Perforation in einer Membran (10), aufweisend eine proximale Seite und eine distale Seite, die Membranverschlussvorrichtung (100) umfassend:

   ein Pflaster (120), das selektiv von einer schmalen zu einer erweiterten Konfiguration erweiterbar ist und in der erweiterten Konfiguration eine konkave Oberfläche (122) aufweist, die einen Rückhaltehohlraum (150) zur Aufnahme von Klebstoff (140) definiert;
   einen Pflaster-Zuführmechanismus (110) zum Leiten des Pflasters (120) durch eine Perforation der Membran (10) von einer proximalen zu einer distalen Seite davon;
   einen Befestigungsmittel-Zuführmechanismus (130, 330), der operativ Klebstoff enthält (140) und weiterhin operativ den darin enthaltenen Klebstoff (140) von der proximalen Seite zur distalen Seite der Membran (10) zuführt, zum Befestigen des Pflasters (120) in der erweiterten Konfiguration durch den Klebstoff (140) an der distalen Seite der Membran (10), zum Abdichten der Perforation
   **dadurch gekennzeichnet, dass**
   der Rückhaltehohlraum (150) ein Volumen definiert, das größer ist als das Volumen des Klebstoffs (140), der zu jedem beliebigen Zeitpunkt von dem Befestigungsmittel-Zuführmechanismus (130, 330) enthalten sein kann.

2. Die Membranverschlussvorrichtung (100) gemäß Anspruch 1, umfassend mindestens einen Schlauch, aufweisend ein distales und ein proximales Ende, zum Befördern des Pflasters (120) in einer gefalteten Konfiguration durch den mindestens einen Schlauch von dem distalen zu dem proximalen Ende, zum Befestigen des Pflasters (120) in einer erweiterten Konfiguration an einer Innenfläche (16) der Membran (10).

3. Die Membranverschlussvorrichtung (100) gemäß einem der vorhergehenden Ansprüche, wobei das Pflaster (120) ein selbst-erweiterbares Pflaster (120) ist.

4. Die Membranverschlussvorrichtung (100) gemäß Anspruch 3, wobei das selbsterweiterbare Pflaster (120) eine schirmartige Vorrichtung ist, umfassend eine Stützstruktur (123A, 123B), die Streben und eine Verschlussfolie (121) enthält, die an den Streben befestigt ist.

5. Die Membranverschlussvorrichtung (100) gemäß Anspruch 4, wobei die Streben mindestens zwei Drähte (124A, 124B) umfassen, die in der erweiterten Konfiguration eine S-förmige Form oder die Form oder Kontur einer blättrigen Blume annehmen.

6. Die Membranverschlussvorrichtung (100) gemäß Anspruch 5, wobei die Streben ein Formgedächtnismaterial umfassen, das beispielsweise aus Nitinol besteht.

7. Die Membranverschlussvorrichtung (100) gemäß einem der vorhergehenden Ansprüche, wobei der Klebstoff (140) einen biokompatiblen Adhäsiv, ein biokompatibles Versiegelungsmittel, ein versiegelungsinduzierendes Material und/oder ein heilungsinduzierendes Material umfasst.

8. Die Membranverschlussvorrichtung (100) gemäß einem der vorhergehenden Ansprüche, wobei der Befestigungsmittel-Zuführmechanismus (130, 330) einen Mischer (360) umfasst, der in einem schlauchförmigen Körper (111) des Geräts zum Mischen von Komponenten zur Herstellung eines Adhäsivs enthalten ist.

9. Die Membranverschlussvorrichtung (100) gemäß Anspruch 8, wobei der Mischer (360) einen Mischkörper (361) und Rippen (363) umfasst, wobei die Rippen (363) nach innen in den Mischkörper (361) hineinragen, zum Mischen unterschiedlicher Adhäsiv-Zusammensetzungen.

10. Die Membranverschlussvorrichtung (100) gemäß einem der vorhergehenden Ansprüche umfasst einen Pflaster-Entkopplungsmechanismus zum Lösen des Pflasters (120) von der Membranverschlussvorrichtung nach Befestigung des Pflasters (120) an der Innenfläche (16) einer Perforationsstelle (12) der Membran (10).

11. Die Membranverschlussvorrichtung (100) gemäß einem der vorhergehenden Ansprüche, wobei der Klebstoff (140) einen aus folgenden umfasst: einen Wachstumsfaktor und/oder ein zellinstruktives Gerüst, wie zum Beispiel eine dezellularisierte Amnionmembran.

12. Die Membranverschlussvorrichtung (100) gemäß einem der vorhergehenden Ansprüche zur Verwendung bei der Behandlung einer Perforation einer fetalen Membran, zur Verwendung bei der Behandlung einer Perforation einer vaskulären Membran, zur Verwendung bei der Behandlung einer Perforation einer kardiovaskulären Membran und/oder zur Verwendung bei der Behandlung einer Trommelfell-Membran.

**Revendications**

1. Dispositif de fermeture de membrane (100) pour fermer une perforation dans une membrane (10) ayant un côté proximal et un côté distal, le dispositif de fermeture de membrane (100) comprenant :

   un patch (120) qui peut être dilaté sélectivement d'une configuration étroite à une configuration dilatée, et ayant dans la configuration dilatée une surface concave (122) définissant une cavité de rétention (150) pour recevoir de la colle (140) ;
   un mécanisme de distribution de patch (110) pour guider le patch (120) à travers une perforation de la membrane (10) d'un côté proximal à un côté distal de celle-ci ;
   un mécanisme de distribution de matériel de fixation (130, 330) pouvant être opéré pour contenir de la colle (140) et pouvant en outre être opéré pour distribuer la colle (140) contenue en son sein du côté proximal au côté distal de la membrane (10) pour fixer le patch (120) dans la configuration dilatée, par la colle (140), au côté distal de la membrane (10) pour sceller la perforation,
   **caractérisé en ce que**
   la cavité de rétention (150) définit un volume qui est supérieur à un volume de colle (140) qui peut être contenu, à tout moment donné, par le mécanisme de distribution de matériel de fixation (130, 330).

2. Dispositif de fermeture de membrane (100) selon la revendication 1, comprenant au moins un tube ayant une extrémité distale et une extrémité proximale pour distribuer le patch (120) dans une configuration pliée par le biais de l'au moins un tube de l'extrémité distale à l'extrémité proximale pour relier le patch (120) dans une configuration dilatée à une surface interne (16) de la membrane (10).

3. Dispositif de fermeture de membrane (100) selon l'une quelconque des revendications précédentes, dans lequel le patch (120) est un patch auto-expansible (120).

4. Dispositif de fermeture de membrane (100) selon la revendication 3, dans lequel le patch auto-expansible (120) est un dispositif de type parapluie comprenant une structure de support (123A, 123B) incluant des montants et une feuille de fermeture (121) fixée aux montants.

5. Dispositif de fermeture de membrane (100) selon la revendication 4, dans lequel les montants comprennent au moins deux fils métalliques (124A, 124B) qui adoptent, dans la configuration dilatée, une forme de S ou la forme ou le contour d'une fleur à pétales.

6. Dispositif de fermeture de membrane (100) selon la revendication 5, dans lequel les montants comprennent du matériau à mémoire de forme composé, par exemple, de Nitinol.

7. Dispositif de fermeture de membrane (100) selon l'une quelconque des revendications précédentes, dans lequel la colle (140) comprend un adhésif biocompatible, un produit d'étanchéité biocompatible, un matériau inducteur d'étanchéité et/ou un matériau inducteur de cicatrisation.

8. Dispositif de fermeture de membrane (100) selon l'une quelconque des revendications précédentes, dans lequel le mécanisme de distribution de matériel de fixation (130, 330) comprend un mixeur (360) qui est inclus dans un corps en forme de tube (111) du dispositif pour mélanger des composants pour produire un adhésif.

9. Dispositif de fermeture de membrane (100) selon la revendication 8, dans lequel le mixeur (360) comprend un corps de mélange (361) et des ailettes (363), dans lequel les ailettes (363) font saillie vers l'intérieur dans le corps de mélange (361) pour mélanger différente matière adhésive de composition.

10. Dispositif de fermeture de membrane (100) selon l'une quelconque des revendications précédentes, comprenant un mécanisme de déconnexion de patch pour libérer le patch (120) du dispositif de fermeture de membrane à la suite de la fixation du patch (120) à la surface interne (16) d'un site de perforation (12) de la membrane (10).

**11.** Dispositif de fermeture de membrane (100) selon l'une quelconque des revendications précédentes, dans lequel la colle (140) comprend l'un quelconque des éléments suivants : un facteur de croissance et/ou un échafaudage instructif cellulaire comme, par exemple, une membrane amniotique décellularisée.

**12.** Dispositif de fermeture de membrane (100) selon l'une quelconque des revendications précédentes destiné à être utilisé dans le traitement d'une perforation d'une membrane foetale, destiné à être utilisé dans le traitement d'une perforation d'une membrane vasculaire, destiné à être utilisé dans le traitement d'une perforation d'une membrane cardiovasculaire et/ou destiné à être utilisé dans le traitement d'une membrane tympanique.

FIG. 1

FIG. 2A

FIG. 2B

FIG. 2C

FIG. 2D

FIG. 2E

FIG. 2F

FIG. 2G

EP 3 576 637 B1

FIG. 2H

FIG. 3A

FIG. 3B

FIG. 3C

330/314

331A  331B

350

120

FIG. 4A

350

341A  341B

FIG. 4B

EP 3 576 637 B1

FIG. 5

FIG. 6B

FIG. 6A

710 DEPLOY AT LEAST ONE EXPANDABLE PATCH TO A PERFORATION SITE

720 DELIVER A FASTENER TO THE PERFORATION SITE

730 FASTEN THE AT LEAST ONE EXPANDABLE PATCH IN AN EXPANDED CONFIGURATION, BY THE FASTENER, TO THE MEMBRANE TO SEAL THE PERFORATION

FIG. 7

EP 3 576 637 B1

FIG. 8A

FIG. 8B

FIG. 8C

FIG. 8D

FIG. 9E

FIG. 9F

FIG. 9C

FIG. 9D

FIG. 9B

FIG. 9A

FIG. 10C

FIG. 10D

FIG. 10B

FIG. 10A

A and B:
Scale bar: 5 mm

C:
Scale bar: 2 cm

FIG. 11

Scale bar: 5 cm

FIG. 12

Scale bar: 5 mm

n=5; **: p<0.02

FIG. 13

**:p < 0.02

scale bars = 5mm

FIG. 14

FIG. 15

Scale bar: 5 mm

## REFERENCES CITED IN THE DESCRIPTION

### Patent documents cited in the description

- EP 3300669 A

- US 2008058710 A1

### Non-patent literature cited in the description

- **LEE et al.** *Nature,* 2007, vol. 448, 338-41
- **BILIC et al.** *American Journal of Obstetrics and Gynecology,* 2010, vol. 202 (85), e1-9
- **HALLER et al.** *Acta biomaterialia,* 2012, vol. 8, 4365-70
- **OCHSENBEIN-KOLBLE, N et al.** Enhancing sealing of fetal membrane defects using tissue engineered native amniotic scaffolds in the rabbit model. *American journal of obstetrics and gynecology,* 2007, vol. 196 (263), e261-267
- **BRUBAKER, C.E. ; KISSLER, H. ; WANG, L.J ; KAUFMAN, D.B ; MESSERSMITH, P.B.** Biological performance of mussel-inspired adhesive in extrahepatic islet transplantation. *Biomaterials,* 2010, vol. 31, 420-427

- **BURKE, S.A. ; RITTER-JONES, M ; LEE, B.P. ; MESSERSMITH, P.B.** Thermal gelation and tissue adhesion of biomimetic hydrogels. *Biomedical materials,* 2007, vol. 2, 203-210
- **LEE, B.P. ; DALSIN, J.L ; MESSERSMITH, P.B.** Synthesis and gelation of DOPA-modified poly(ethylene glycol) hydrogels. *Biomacromolecules,* 2002, vol. 3, 1038-1047
- **LEE, H. ; LEE, B.P ; MESSERSMITH, P.B.** A reversible wet/dry adhesive inspired by mussels and geckos. *Nature,* 2007, vol. 448, 338-341
- **LEE, H ; SCHERER, N.F. ; MESSERSMITH, P.B.** Single-molecule mechanics of mussel adhesion. *Proc Natl Acad Sci U S A,* 2006, vol. 103, 12999-13003